# EUROPEAN PATENT APPLICATION

(11) **EP 3 251 580 A1**
(43) Date of publication of application: **06.12.2017**
(21) Application number: 16743133.7
(22) Date of filing: 15.01.2016
(51) Int. Cl.: A61B 1/00, G02B 23/24

(54) **ENDOSCOPE**

(30) Priority: 26.01.2015 JP 2015012528
(71) Applicant: Olympus Corporation, Tokyo 192-8507 (JP)
(72) Inventor: HOSHINO, Yuki, Tokyo 192-8507 (JP); FUNAKOSHI, Yasuo, Tokyo 192-8507 (JP); FUKUDA, Hiroyuki, Tokyo 192-8507 (JP); SUGAYA, Kota, Tokyo 192-8507 (JP)
(74) Representative: Schicker, Silvia
(86) International application number: PCT/JP2016/051118
(87) International publication number: WO 2016/121529

(57) **Abstract**

An endoscope includes a universal cord which is connected to an operation section at one end thereof and which is configured to connect an external device to the operation section; a first switch which is disposed at about the same position as a connecting position to the universal cord along a longitudinal axis of the operation section; and a second switch which is disposed on a side opposite to the insertion section via the first switch along the longitudinal axis of the operation section, and which is formed to be higher than the first switch in a direction perpendicular to the longitudinal axis.

## Description

### Technical Field

This invention relates to an endoscope including an operation section.

### Background Art

For example, in Jpn. Pat. Appln. KOKAI Publication No. 2000-10023, an endoscope is disclosed in which switches are arranged in an operation section held to be operated by an operator. In this operation section of the endoscope, a first switch is disposed adjacent to a fluid control button and a second switch is disposed in a distal portion to an insertion section. These switches are pressed toward an inner portion of the operation section to suitably change functions.

The operator of the endoscope disclosed in Jpn. Pat. Appln. KOKAI Publication No. 2000-10023 might extend, for example, a middle finger or a ring finger to maintain a position of a turning knob that bends a bending section. Depending on a size of a hand, a length of an index finger or the like, there might be a case where it is difficult to bend a fingertip of the index finger and operate the second switch in a state where the position of the turning knob is maintained.

### Summary of Invention

An object of this invention is to provide an endoscope having switch which is operable without bending a fingertip of an index finger.

According to one aspect of the present invention, an endoscope includes an operation section which is connected to an insertion section to be inserted into a subject and which is configured to operate the insertion section; a universal cord which is connected to the operation section at one end thereof and which is configured to connect an external device to the operation section; a first switch which is disposed at about the same position as a connecting position to the universal cord along a longitudinal axis of the operation section; and a second switch which is disposed on a side opposite to the insertion section via the first switch along the longitudinal axis of the operation section, and which is formed to be higher than the first switch in a direction perpendicular to the longitudinal axis.

### Brief Description of Drawings

FIG. 1A is a schematic view showing an endoscope according to a first embodiment;
FIG. 1B is a schematic view showing an operation section of the endoscope according to the first embodiment which is seen from a direction of an arrow 1B in FIG. 1A;
FIG. 1C is a schematic view showing the operation section of the endoscope according to the first embodiment which is seen from a direction of an arrow 1C in FIG. 1B;
FIG. 1D is a schematic view showing the operation section of the endoscope according to the first embodiment which is seen from a direction of an arrow 1D in FIG. 1A and FIG. 1C;
FIG. 1E is a schematic view showing the operation section of the endoscope according to the first embodiment which is seen from a direction of an arrow 1E in FIG. 1A;
FIG. 2A is a schematic view showing a state of pressing a press portion of a second switch toward an inner portion of the operation section with a finger pad of an index finger while holding the operation section shown in FIG. 1B of the endoscope according to the first embodiment with an operator' s left hand;
FIG. 2B is a schematic view showing a state of pressing the press portion of the second switch toward the inner portion of the operation section with the finger pad of the index finger while holding the operation section shown in FIG. 1C of the endoscope according to the first embodiment with the operator's left hand;
FIG. 3A is a schematic view showing a state of pressing the press portion of the second switch from a side close to an insertion section toward a side away therefrom with a fingertip of the index finger while holding the operation section shown in FIG. 1B of the endoscope according to the first embodiment with the operator's left hand;
FIG. 3B is a schematic view showing a state of pressing the press portion of the second switch from the side close to the insertion section toward the side away therefrom with the fingertip of the index finger while holding the operation section shown in FIG. 1C of the endoscope according to the first embodiment with the operator's left hand;
FIG. 4 is a schematic view showing a state of an operation section of an endoscope according to a modification of the first embodiment which is seen from the same direction as in FIG. 1B;
FIG. 5 is a schematic view showing a state of the operation section of the endoscope according to the modification of the first embodiment which is seen from the same direction as in FIG. 1A;
FIG. 6 is a schematic view showing a state of placing a finger pad of a thumb on a press portion of a fourth switch and placing a finger pad of an index finger on a press portion of a second switch while holding the operation section shown in FIG. 1C of the endoscope according to the modification of the first embodiment with the operator's left hand;
FIG. 7A is a schematic view showing an operation section of an endoscope according to a second embodiment;
FIG. 7B is a schematic view showing the operation section of the endoscope according to the second embodiment which is seen from a direction of an arrow 7B in FIG. 7A;
FIG. 7C is a schematic view showing the operation section of the endoscope according to the second embodiment which is seen from a direction of an arrow 7C in FIG. 7B;
FIG. 7D is a schematic view showing the operation section of the endoscope according to the second embodiment which is seen from a direction of an arrow 7D in FIG. 7A and FIG. 7C; and
FIG. 7E is a schematic view showing the operation section of the endoscope according to the second embodiment which is seen from a direction of an arrow 7E in FIG. 7A.

### Description of Embodiments

Hereinafter, embodiments of this invention will be described with reference to the drawings.

### [First Embodiment]

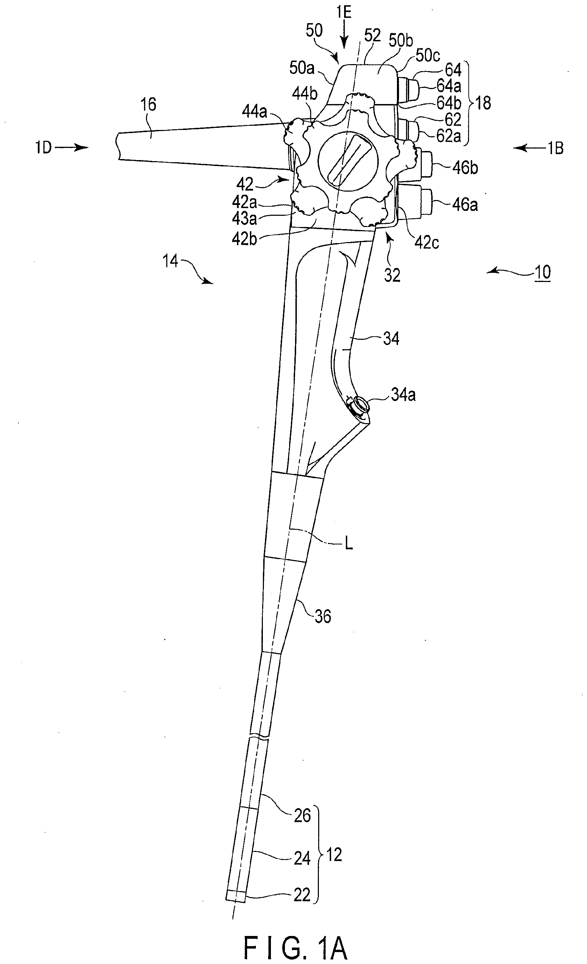

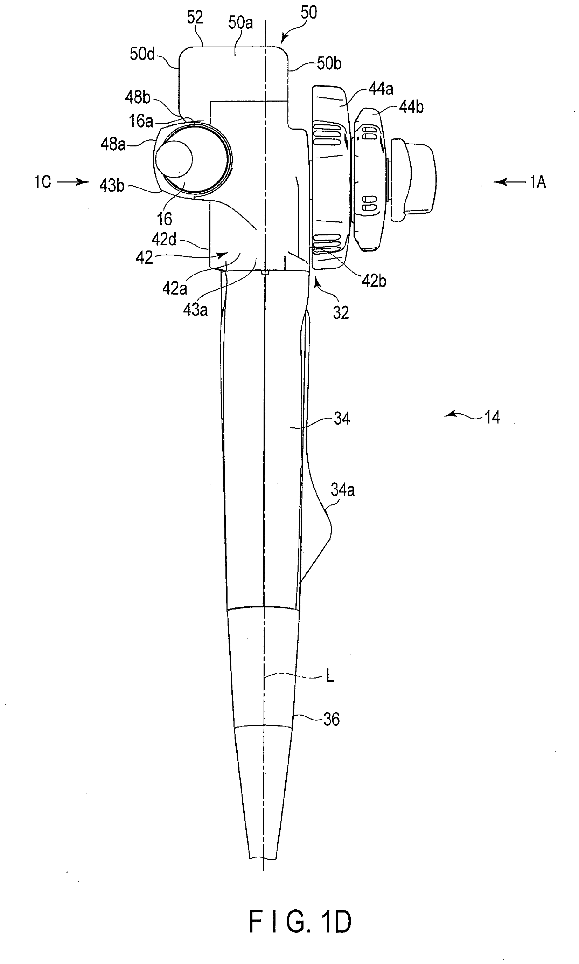

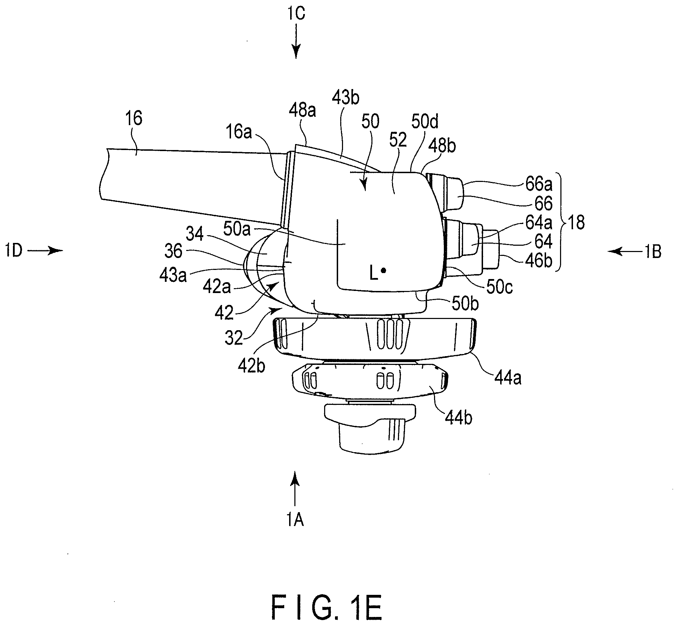

A first embodiment will be described with reference to FIG. 1A to FIG. 3B.

As shown in FIG. 1A, an endoscope 10 according to this embodiment includes an insertion section 12 to be inserted into a subject, an operation section 14 held by an operator to operate the insertion section 12, a universal cord 16 disposed in the operation section 14 at one end thereof and connected to an external device such as a control device of the endoscope 10, and a switch section 18. The universal cord 16 connects the external device and the operation section 14. Although not shown in the drawing, the endoscope 10 includes an illumination optical system and an observation optical system as it is known. The illumination optical system and the observation optical system are disposed from a distal portion of the insertion section 12 of the endoscope 10 through the operation section 14 to an unshown connector of the universal cord 16.

As it is known, the insertion section 12 includes a distal hard portion 22, a bending section 24 and a flexible tubular portion 26 in order from its distal end to its proximal end. The proximal end of the insertion section 12, that is, a proximal end of the flexible tubular portion 26 is connected to the operation section 14.

As shown in FIG. 1A to FIG. 1E, the operation section 14 includes a main body 32 having various mechanisms to be operated, a holding portion 34 to be held by an operator (a user), and an anti-break 36 that prevents buckling of the insertion section 12. In the operation section 14, the anti-break 36, the holding portion 34 and the main body 32 are arranged in this order along its longitudinal axis L in order from a side close to the insertion section 12 toward a side away therefrom. The anti-break 36 is connected to the proximal end of the flexible tubular portion 26 via an unshown mouth ring or the like.

The holding portion 34 is tubularly formed and has a proximal opening 34a of an unshown channel. The proximal opening 34a of the channel is interposed between the main body 32 and the anti-break 36.

The main body 32 includes a first outer case 42, turning knobs 44a and 44b, fluid control buttons 46a and 46b, bulging portions 48a and 48b, and a second outer case 50.

The switch section 18 is disposed in the first and second outer cases 42 and 50 of the operation section 14. In this embodiment, the switch section 18 includes a first switch 62 disposed in the first outer case 42 and second and third switches 64 and 66 disposed in the second outer case 50.

The second outer case 50 is coupled with the first outer case 42 at a position away from the insertion section 12 along the longitudinal axis L of the operation section 14. The second outer case 50 is separable to the first outer case 42 during maintenance. Consequently, there is easy maintenance such as replacement of the second and third switches 64 and 66 disposed in the second outer case 50. It is to be noted that the second outer case 50 is separated from the first outer case 42, so that the maintenance of the first switch 62 can easily be performed in a state where the second outer case 50 is detached from the first outer case 42.

The first outer case 42 schematically includes first to fourth surfaces 42a, 42b, 42c and 42d along a periaxial direction of the longitudinal axis L of the operation section 14. The first outer case 42 forms a tubular outer shell by the first to fourth surfaces 42a, 42b, 42c and 42d. Here, the first to third surfaces 42a, 42b and 42c are integrated, and the fourth surface 42d including the first and second bulging portions 48a and 48b forms a lid on an opening formed by integrating the first to third surfaces 42a, 42b and 42c. That is, the first outer case 42 includes a base body (a first cover body) 43a that is formed by the first to third surfaces 42a, 42b and 42c and has a substantially C-shaped cross section of the longitudinal axis L of the operation section 14, and a lid portion (a second cover body) 43b that is formed by the fourth surface 42d, holds the universal cord 16 and forms a lid on the base body 43a. Consequently, it is easy to perform maintenance of various mechanisms including cables, e.g., a mechanism that is disposed in the first outer case 42 to bend the bending section 24, a mechanism to feed air, feed water and suck from the distal hard portion 22, and a further mechanism that transmits a signal of an observation image of the endoscope 10. These mechanisms are known and hence description thereof is omitted here.

The first surface 42a of the first outer case 42 is adjacent to a proximal end of the universal cord 16 extended out from the operation section 14 in a peripheral direction of the longitudinal axis L of the operation section 14. A thenar of a palm of the operator's left hand is touched to the first surface 42a. It is to be noted that the first surface 42a may face the operator, when the operator holds the operation section 14 with the left hand.

The second surface 42b is formed between the first surface 42a and the third surface 42c along the periaxial direction of the longitudinal axis L of the operation section 14. The second surface 42b may be a right surface to the operator, when the operator holds the operation section 14 with the left hand. In the second surface 42b, there are disposed the turning knobs 44a and 44b which bend the bending section 24 of the insertion section 12. The turning knobs 44a and 44b are turnable in a periaxial direction of a turning center axis of the same axis. The first turning knob 44a is operated with the thumb of the operator's left hand when bending the bending section 24 in an upward direction and a downward direction. The second turning knob 44b is operated with the thumb of the operator's left hand when bending the bending section 24 in a left direction and a right direction.

The third surface 42c is a surface opposite to the first surface 42a to the longitudinal axis L. The third surface 42c may be a distal back surface to the operator, when the operator holds the operation section 14 with the left hand. In the third surface 42c, there are disposed the fluid control buttons 46a and 46b and the first switch 62 of the switch section 18. Consequently, the third surface 42c of the first outer case 42 is formed as a part of the switch section 18. The fluid control buttons 46a and 46b are arranged to be adjacent along the longitudinal axis L of the operation section 14. The fluid control buttons 46a and 46b are disposed on a side closer to the insertion section 12 than the first switch 62 along the longitudinal axis L of the operation section 14. The first fluid control button 46a is for use in feeding air to discharge the air (a gas) from a distal end of the insertion section 12 by an operation of closing a hole and in feeding water to discharge a physiological saline solution or the like from the distal end of the insertion section 12 by a pressing operation. The second fluid control button 46b is for use in sucking a liquid such as blood or the physiological saline solution or a solid such as a living tissue from the distal end of the insertion section 12 by the pressing operation.

As the first switch 62, a suitable switch is used in which a press portion 62a is pressed toward an inner portion of the operation section 14 to suitably change a function.

The first switch 62 is for use in freezing the observation image of the endoscope 10 which is displayed in, for example, an unshown monitor. To the first switch 62, there is assigned a suitable function such as a function of suitably controlling the observation optical system in the endoscope 10. It is also preferable to assign a function of suitably controlling the illumination optical system to the first switch 62.

The fourth surface 42d is formed between the third surface 42c and the first surface 42a along the periaxial direction of the longitudinal axis L of the operation section 14. The fourth surface 42d may be a left surface to the operator, when the operator holds the operation section 14 with the left hand. In the fourth surface 42d, there is formed the first bulging portion 48a that holds the proximal end of the universal cord 16. The first bulging portion 48a is formed to bulge out toward a side opposite to the surface in which the turning knobs 44a and 44b are disposed along the longitudinal axis L. The universal cord 16 is extended out from the first bulging portion 48a substantially in parallel with a normal direction of the first surface 42a. In the first bulging portion 48a, its bulging amount on a side close to the third surface 42c is smaller than its bulging amount on a side close to the first surface 42a. Consequently, due to this difference in bulging amount, a side of the first bulging portion 48a which is close to the insertion section 12 is formed as an inclined surface. In the inclined surface, a side close to the third surface 42c is away from the insertion section 12 along the longitudinal axis L of the operation section 14 as compared with a side close to the first surface 42a. When the operator holds the operation section 14, the vicinity of a root of the index finger of the operator's left hand abuts on a side of the first bulging portion 48a which is close to the insertion section 12, and a portion of the palm of the operator's left hand which is close to a region from the index finger to a little finger is touched to the fourth surface 42d.

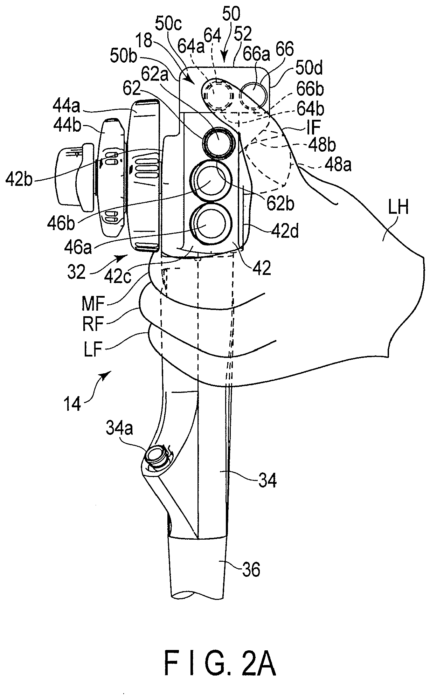

In the first outer case 42 of the main body 32, the first surface 42a is continuous with the holding portion 34 without any stepped portions. It is preferable that in the first outer case 42, the second surface 42b is continuous with the holding portion 34 without any stepped portions. In the first outer case 42, the third surface 42c and the fourth surface 42d project to the holding portion 34 and are formed with the stepped portions. Consequently, when the operator of the endoscope 10 holds the operation section 14 with a left hand LH as shown in FIG. 2A to FIG. 3B, for example, a middle finger MF is hooked on the stepped portions among the third surface 42c and the fourth surface 42d and the holding portion 34. The fluid control buttons 46a and 46b and the press portion 62a of the first switch 62 are suitably operable with, for example, a finger pad of an index finger IF. Further, when the operator of the endoscope 10 holds the operation section 14 with the left hand LH, the universal cord 16 is extended out on the left side of the operator. In this way, the fluid control buttons 46a and 46b and the first switch 62 are formed in a surface of the main body 32 of the operation section 14 which is opposite to an extending direction of the universal cord 16 extended out from the operation section 14.

In the fourth surface 42d of the first outer case 42, at a position away from the insertion section 12, there is formed the second bulging portion 48b that is smoothly continuous with after-mentioned third continuous surface 50c and fourth continuous surface 50d of the second outer case 50. The second bulging portion 48b is formed at a position of the fourth surface 42d which is close to the third surface 42c and away from the first surface 42a. The second bulging portion 48b does not project out to the third surface 42c, but projects out to the fourth surface 42d.

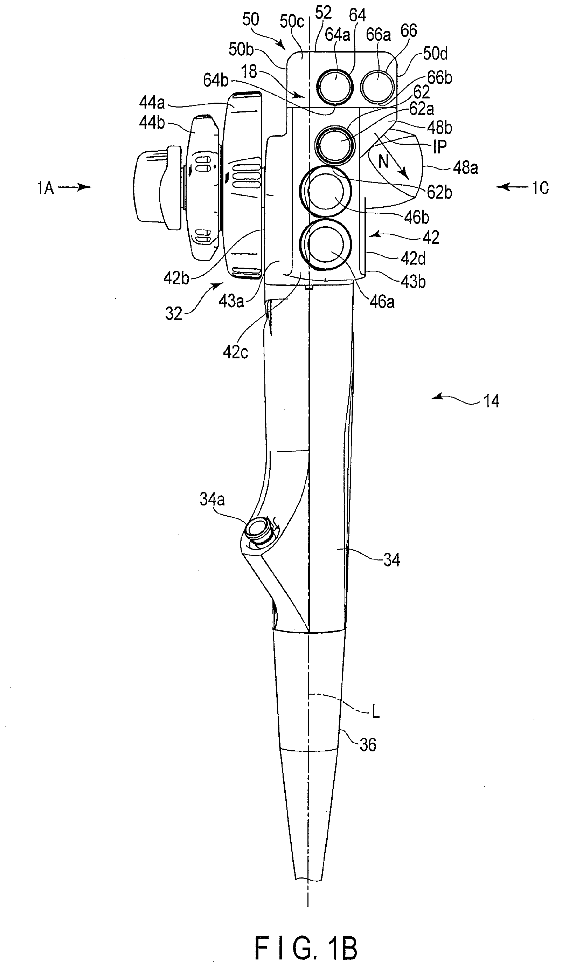

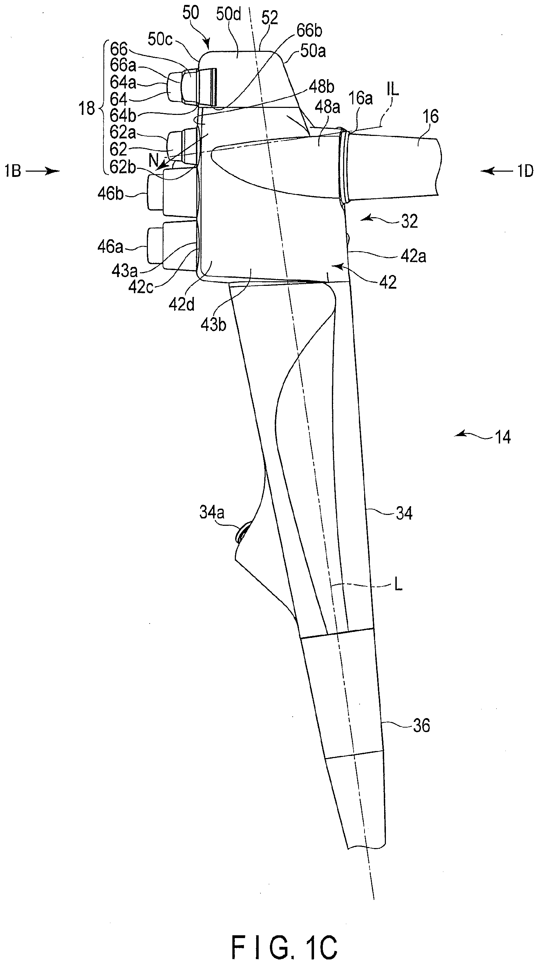

The second bulging portion 48b is formed as an inclining curved surface so that the third surface 42c is smoothly continuous with the fourth surface 42d in the first outer case 42. A normal N of the second bulging portion 48b shown in FIG. 1B and FIG. 1C is stipulated toward a side close to the insertion section 12, toward the third surface 42c and toward a side away from the fourth surface 42d. Consequently, the second bulging portion 48b also functions as a guide surface that does not disturb the index finger IF to be placed on the fluid control buttons 46a and 46b and the first switch 62 and that is easy to guide the index finger IF to the second switch 64 and the third switch 66.

As shown in FIG. 1B, in this embodiment, the second bulging portion 48b is formed in a state of having an inflection point IP so that the fourth surface 42d is smoothly continuous with the after-mentioned fourth continuous surface 50d of the second outer case 50. The second bulging portion 48b cooperates with the third surface 42c and the fourth surface 42d of the first outer case 42 to be continuous with the third continuous surface 50c and the fourth continuous surface 50d of the second outer case 50 without any stepped portions.

The second outer case 50 includes a first continuous surface 50a that is continuous with the first surface 42a of the first outer case 42, a second continuous surface 50b that is continuous with the second surface 42b, the third continuous surface 50c that is continuous with the third surface 42c, and the fourth continuous surface 50d that is continuous with the fourth surface 42d. The second outer case 50 further includes an end face (a top face) 52 away from the insertion section 12. The first to fourth continuous surfaces 50a, 50b, 50c and 50d and the end face 52 form an outer shell of the second outer case 50.

In the third continuous surface 50c, there are disposed the second and third switches 64 and 66 of the switch section 18. That is, the third continuous surface 50c is formed as a part of the switch section 18. In this embodiment, the switch section 18 has one or plural switches (three switches here) 62, 64 and 66. The third continuous surface 50c in which the second and third switches 64 and 66 are disposed is continuous with the third surface 42c in which the first switch 62 is disposed without any stepped portions.

To the second switch 64, there is assigned a suitable function such as a function of emphasizing an image displayed in the unshown monitor. To the third switch 66, there is assigned a suitable function such as a function of releasing the image displayed in the monitor. It is also preferable that the function of the second switch 64 is replaced with that of the third switch 66. Additionally, it is also preferable that the functions of the second and third switches 64 and 66 are assigned to suitably control the illumination optical system, not the observation optical system, thereby changing white light observation and narrow band light observation.

As shown in FIG. 1A to FIG. 1C, the first and second switches 62 and 64 are arranged to be adjacent along the longitudinal axis (a central axis of the insertion section 12) L of the operation section 14. In these switches, the first switch 62 is disposed in the third surface 42c of the first outer case 42. Especially, the first switch 62 is disposed at about the same position as a connecting position of the universal cord 16 to the operation section 14 along the longitudinal axis L. The second switch 64 is formed in the third continuous surface 50c that is continuous with the third surface 42c along the longitudinal axis L of the operation section 14 in the second outer case 50. That is, the second switch 64 is interposed between the third surface 42c of the first outer case 42 and the end face 52 of the second outer case 50. Consequently, the first switch 62 is closer to the fluid control buttons 46a and 46b than the second switch 64, and the second switch 64 is present at a position more away from the insertion section 12 than the first switch 62, i.e., on a side opposite to the insertion section 12 via the first switch 62. Furthermore, the second switch 64 is not disposed in the end face 52, and hence the first and second switches 62 and 64 are present in such a range that the switches are operable by moving a fingertip of the index finger IF in a state where the operation section 14 is held.

The second and third switches 64 and 66 are arranged to be adjacent along the periaxial direction of the longitudinal axis L of the operation section 14. Consequently, the third switch 66 is not disposed in the end face 52, and hence the first to third switches 62, 64 and 66 are present in such a range that the switches are operable by moving the fingertip of the index finger in the state where the operation section 14 is held by the operator. It is to be noted that as shown in FIG. 1B, a center of a press portion 66a of the third switch 66 is present at a position more away from the longitudinal axis L than the fourth surface 42d. Consequently, it is possible to operate the third switch 66 without interfering with the first outer case 42 by the index finger IF as much as possible.

As shown in FIG. 1B and FIG. 1C, it is preferable that the same type of switches are used in the second switch 64 and the third switch 66. Due to the second bulging portion 48b of the first outer case 42 and the third and fourth continuous surfaces 50c and 50d of the second outer case 50, a position of a base portion of the second and third switches 64 and 66 shifts in a direction perpendicular to the longitudinal axis L of the operation section 14 (a direction in which the universal cord 16 extends). Consequently, it is possible to form a difference in position (height) between a press portion 64a of the second switch 64 and the press portion 66a of the third switch 66. That is, the press portion (a top portion) 64a of the second switch 64 projects to the third surface 42c more than the press portion (a top portion) 66a of the third switch 66. It is preferable that the press portion 64a of the second switch 64 projects, for example, as much as a thickness of the index finger, more specifically as much as approximately 5 mm to about 8 mm, as compared with the press portion 66a of the third switch 66.

Here, as shown in FIG. 1C, there is drawn an imaginary line IL that passes a position (a position at which the universal cord 16 is disposed) 16a of the proximal end of the universal cord 16 which is most away from the insertion section 12 along the longitudinal axis L of the operation section 14 and that is perpendicular to the longitudinal axis L. At this time, in the second switch 64, an edge portion 64b closest to the insertion section 12 along the longitudinal axis L of the operation section 14 is disposed on a side of the operation section 14 which is more away from the insertion section 12 than the imaginary line IL, on the basis of the imaginary line IL, i.e., the position 16a of the universal cord 16 which is most away from the insertion section 12 along the longitudinal axis L of the operation section 14. In the third switch 66, an edge portion 66b closest to the insertion section 12 along the longitudinal axis L of the operation section 14 is disposed on a side of the operation section 14 which is more away from the insertion section 12 than the imaginary line IL, on the basis of the imaginary line IL, i.e., the position 16a of the universal cord 16 which is most away from the insertion section 12 along the longitudinal axis L of the operation section 14.

Additionally, it is preferable that in the first switch 62, an edge portion 62b closest to the insertion section 12 is present on a side closer to the insertion section 12 than the imaginary line IL perpendicular to the longitudinal axis L, on the basis of the position 16a of the proximal end of the universal cord 16 which is most away from the insertion section 12.

As shown in FIG. 2A to FIG. 3B, as the second switch 64, a suitable switch is used in which the press portion 64a is pressed to suitably change the function. In the second switch 64, the press portion 64a is pressed toward the inner portion of the operation section 14 to suitably change the function. In the second switch 64, the press portion 64a is pressed from a side close to the insertion section 12 toward a side away therefrom to suitably change the function. Alternatively, in the second switch 64, the press portion 64a is pressed from the side close to the insertion section 12 toward the side away therefrom and in the periaxial direction of the longitudinal axis L of the operation section 14 to suitably change the function. In this way, the second switch 64 can be pushed from the side close to the insertion section 12 toward the side away therefrom. Especially, the second switch 64 can be pushed from the side close to the insertion section 12 toward the side away therefrom and from the fourth continuous surface 50d toward the second continuous surface 50b in the periaxial direction of the longitudinal axis L of the operation section 14.

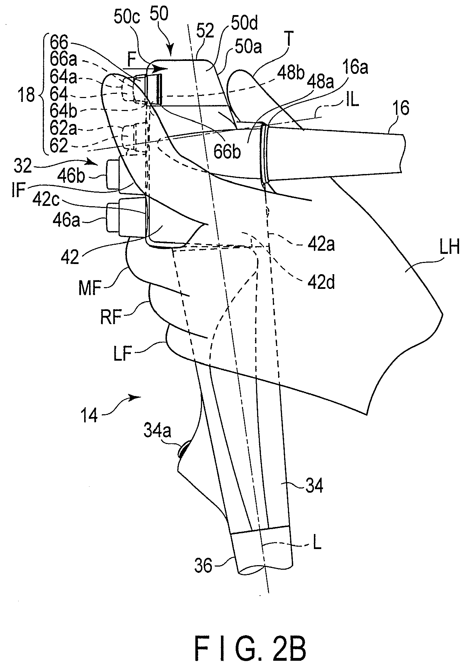

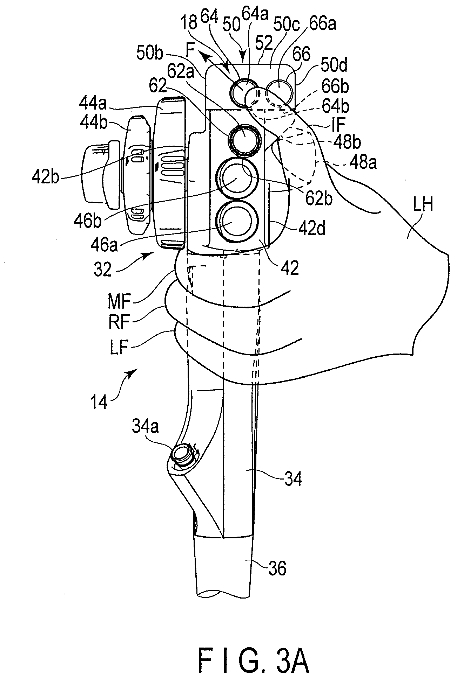

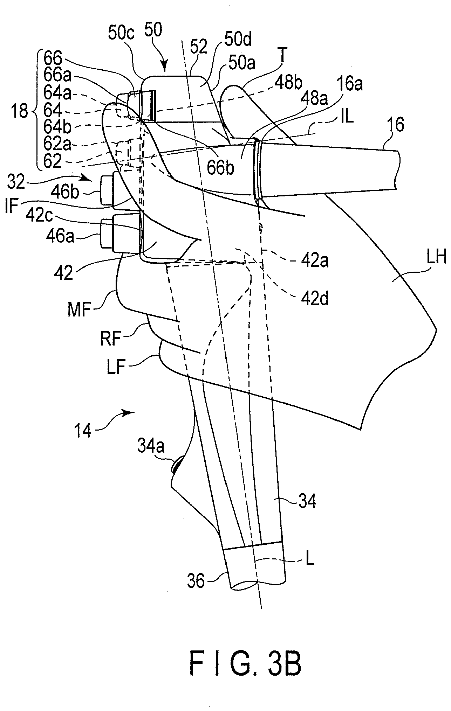

Consequently, as shown in FIG. 2A and FIG. 2B, in the second switch 64, the press portion 64a can be pressed toward the inner portion of the operation section 14 by, for example, the finger pad of the index finger IF of the left hand LH of the operator who has comparatively large hands. As shown in FIG. 3A and FIG. 3B, in the second switch 64, the press portion 64a can be pressed from the side close to the insertion section 12 toward the side away therefrom by, for example, a fingertip of the index finger IF of the left hand LH of the operator who has, for example, comparatively small hands. In the second switch 64, the press portion 64a can be pressed from the side close to the insertion section 12 toward the side away therefrom and from the fourth continuous surface 50d toward the second continuous surface 50b in the periaxial direction of the longitudinal axis L of the operation section 14 by, for example, the index finger IF of the left hand LH of the operator who has comparatively small hands. That is, a force may be given to the second switch 64 to press the second switch in the periaxial direction of the longitudinal axis L of the operation section 14, and the force can be given to press the second switch toward the inner portion of the operation section 14.

In this way, as the second switch 64, a suitable switch is used in which the press portion 64a is pressed toward the inner portion of the operation section 14 to suitably change the function and in which the press portion 64a is pressed from the side close to the insertion section 12 toward the side away therefrom to suitably change the function.

In the third switch 66, the edge portion 66b closest to the insertion section 12 along the longitudinal axis L of the operation section 14 is disposed at a position of the operation section 14 which is more away from the insertion section 12 than the position 16a of the universal cord 16 which is most away from the insertion section 12 along the longitudinal axis L of the operation section 14.

In the third switch 66, the edge portion 66b closest to the insertion section 12 along the longitudinal axis L of the operation section 14 is disposed on a side of the operation section 14 which is more away from the insertion section 12 than the imaginary line IL, on the basis of the imaginary line IL, i.e., a position of the universal cord 16 which is away from the insertion section 12 along the longitudinal axis L of the operation section 14.

The third switch 66 can be pushed from the side close to the insertion section 12 toward the side away therefrom. Especially, the third switch 66 can be pushed from the side close to the insertion section 12 toward the side away therefrom, and can be pushed toward the longitudinal axis L of the operation section 14, i.e., toward the inner portion of the operation section 14.

As the third switch 66, a suitable switch is used so that the press portion 66a is pressed toward the inner portion of the operation section 14 to suitably change the function and so that the press portion 66a is pressed from the side close to the insertion section 12 toward the side away therefrom to suitably change the function. Consequently, in the third switch 66, the press portion 66a can be pressed toward the inner portion of the operation section 14 with the index finger IF of the operator who has, for example, comparatively large hands. In the third switch 66, the press portion 66a can be pressed from the side close to the insertion section 12 toward the side away therefrom with the index finger IF of the left hand of the operator who has, for example, comparatively small hands.

In this way, as the third switch 66, the suitable switch is used in which the press portion 66a is pressed toward the inner portion of the operation section 14 to suitably change the function and in which the press portion 66a is pressed from the side close to the insertion section 12 toward the side away therefrom to suitably change the function.

In this embodiment, as shown in FIG. 1A, in the first and second switches 62 and 64 which are adjacent to each other, a height of the press portion 62a of the first switch 62 to the third surface 42c in which the first switch 62 is disposed is about the same as the height (the position) of the press portion 64a of the second switch 64 to the third continuous surface 50c in which the second switch 64 is disposed. In the second and third switches 64 and 66 which are adjacent to each other, the height (the position) of the press portion 64a to the third continuous surface 50c of the second outer case 50 is different from that of the press portion 66a. Here, the press portion 64a of the second switch 64 is present at the position higher than the press portion 66a of the third switch 66. In this case, it is preferable that the press portion 64a of the second switch 64 projects, for example, as much as about a half of a thickness (from about 9.9 mm to 15.5 mm) of the index finger, more specifically as much as approximately 4 mm to about 8 mm that is about the half of the thickness of the index finger IF, as compared with the press portion 66a of the third switch 66.

Next, an operation of the endoscope 10 according to this embodiment will be described.

The suitable functions are assigned to the first to third switches 62, 64 and 66. Here, the function of freezing the observation image is assigned to the first switch 62, the function of emphasizing a picture of the observation image is assigned to the second switch 64, and the function of releasing the observation image is assigned to the third switch 66, respectively. Additionally, it is preferable to assign a function of suitably adjusting the illumination optical system to one of the first to third switches 62, 64 and 66.

As shown in FIG. 2A to FIG. 3B, the operator holds the operation section 14 of the endoscope 10 with the left hand LH, and holds the insertion section 12 with a right hand that is not shown in the drawing. The operator places the vicinity of a root between a thumb T and the index finger IF (vicinity of a web) of the left hand LH on the surface of the first bulging portion 48a on the side close to the insertion section 12, holds the holding portion 34 with the palm, the middle finger MF, a ring finger RF and a little finger LF of the left hand LH, and places the index finger IF on the first switch 62 or the fluid control button 46a or 46b. At this time, the first surface 42a touches on the thenar of the palm of the left hand LH and the fourth surface 42d touches on a region between the thenar of the palm and the root of the index finger IF.

The operator holds the insertion section 12 with the right hand to suitably insert the insertion section 12 from its distal end toward its proximal end into a subject (a pipeline) such as a body cavity.

In a case where the operator is to freeze the image displayed in the unshown monitor, the operator presses the press portion 62a of the first switch 62 toward the inner portion of the main body 32 of the operation section 14 with the finger pad of the index finger IF of the left hand LH.

In a case where the operator is to emphasize the image displayed in the unshown monitor, the operator moves the index finger IF of the left hand LH from the press portion 62a of the first switch 62 to the press portion 64a of the second switch 64. At this time, the operator does not have to hold the operation section 14 again (rehold), and may only move the index finger IF. Furthermore, the second switch 64 is present at the position more away from the insertion section 12 than the first switch 62 and the press portion 64a of the second switch 64 is present at the position higher than the press portion 66a of the third switch 66. Consequently, the operator does not have to visually confirm the second switch 64, but can attach the finger pad or fingertip of the index finger IF to the press portion 64a of the second switch 64.

In a case where the operator who has the comparatively large left hand LH presses the press portion 64a of the second switch 64 as shown in FIG. 2A and FIG. 2B, the operator gives a force in a direction of an arrow F in FIG. 2B with the finger pad of the index finger IF. The operator who has the comparatively large hands presses the press portion 64a of the second switch 64 toward the longitudinal axis L of the operation section 14, i.e., toward the inner portion of the operation section 14 with the finger pad of the index finger IF. Consequently, the function of the observation optical system is suitably changed to, e.g., the function of emphasizing the image by the operation of the second switch 64.

In a case where the operator who has the comparatively small left hand LH presses the press portion 64a of the second switch 64 as shown in FIG. 3A and FIG. 3B, the operator gives a force in a direction of an arrow F in FIG. 3A with the fingertip of the index finger IF. The operator who has the comparatively small left hand presses the press portion 64a of the second switch 64 away from the insertion section 12 with the fingertip of the index finger IF. At this time, the operator moves the fingertip of the index finger IF to press the press portion 62a from the side close to the insertion section 12 toward the side away therefrom and in the periaxial direction of the longitudinal axis L of the operation section 14.

Furthermore, there is a case where the operator who has the comparatively large left hand LH extends the index finger IF to press the press portion 64a of the second switch 64 while holding the turning knob 44a with the middle finger MF and/or the ring finger RF. At this time, the operator presses the press portion 64a of the second switch 64 away from the insertion section 12 with the fingertip of the index finger IF. At this time, while holding the turning knob 44a, the operator moves the fingertip of the index finger IF to press the press portion 64a from the side close to the insertion section 12 toward the side away therefrom and in the periaxial direction of the longitudinal axis L of the operation section 14. In this way, when the operator does not easily extend the index finger IF, the operator can give the force to the press portion 64a of the second switch 64 in the direction of the arrow F in FIG. 3A with the fingertip of the index finger IF in the same manner as in the operator who has the comparatively small hands.

In a case where the operator moves the index finger IF of the left hand, for example, from the press portion 62a of the first switch 62 or the press portion 64a of the second switch 64 to the press portion 66a of the third switch 66 or a case where the operator moves the index finger IF from one of the fluid control buttons 46a and 46b and the first switch 62 to the third switch 66 or from the second switch 64 to the third switch 66, the operator does not have to rehold the operation section 14.

The third switch 66 is adjacent to the second switch 64 at the position more away from the insertion section 12 than the first switch 62, and the press portion 66a of the third switch 66 is present at the position lower than the press portion 62a of the first switch 62 and the press portion 64a of the second switch 64. Consequently, even when the operator does not visually confirm the third switch 66, the operator can specify the position of the press portion 66a of the third switch 66.

For example, the operator who has the comparatively large hands presses the press portion 66a of the third switch 66 toward the longitudinal axis L of the operation section 14, i.e., the inner portion of the operation section 14 with the finger pad of the index finger IF.

For example, the operator who has the comparatively small hands presses the press portion 66a of the third switch 66 away from the insertion section 12 with the fingertip of the index finger IF. At this time, the operator moves the fingertip of the index finger IF from the side of the press portion 62a which is close to the insertion section 12 toward the side away therefrom.

It is to be noted that, for example, even when the operator who has the comparatively large hands holds the turning knob 44a with the middle finger MF and/or the ring finger RF and does not easily extend the index finger IF, the operator can give the force to the press portion 66a of the third switch 66 with the fingertip of the index finger IF in the same manner as the operator who has the comparatively small hands.

As described above, the press portion 64a of the second switch 64 projects, for example, as much as the half of the thickness of the index finger IF as compared with the press portion 66a of the third switch 66. In this way, when the difference in height between the press portions 64a and 66a of the second and third switches 64 and 66 is about the half of the thickness of the index finger IF, the operator sufficiently recognizes that the switch is the press portion 64a of the second switch 64 or the press portion 66a of the third switch 66, by touch of the index finger IF, or the like, without visually confirming the press portion of the switch. For example, when the operator tries to touch the press portion 64a of the second switch 64 but moves the index finger IF by mistake toward the press portion 66a of the third switch 66 which is present at the position lower than the press portion 64a of the second switch 64, there is the possibility that the operator misses touching the press portion of the second switch. When the operator tries to touch the press portion 66a of the third switch 66 but moves the index finger IF by mistake toward the press portion 64a of the second switch 64 which is present at the position higher than the press portion 66a of the third switch 66, there is the high possibility that the operator does not touch the top face of the press portion 64a of the second switch 64 but touches a side surface thereof. Furthermore, when the press portion 64a of the second switch 64 is disposed close to the press portion 66a of the third switch 66, there can be the case where the operator simultaneously touches both the press portions. At this time, the operator can identify the switch by the touch. In this way, when the operator tries to operate the press portion 64a of the second switch 64 but touches the press portion 66a of the third switch 66, the operator can recognize that the touched switch is not the switch to be operated. Similarly, when the operator tries to operate the press portion 66a of the third switch 66 but touches the press portion 64a of the second switch 64, the operator can recognize that the touched switch is not the switch to be operated. Consequently, when the press portions 64a and 66a of the second and third switches 64 and 66 are adjacent to each other and especially even when the press portions are disposed close to each other, it is possible to prevent, by the difference in height, wrong operations when the operator operates the press portions 64a and 66a of the second and third switches 64 and 66 with the index finger IF.

As described above, according to the endoscope 10 of this embodiment, it can be considered as follows.

The second switch 64 disposed in the operation section 14 of the endoscope 10 according to this embodiment exerts the function, when the press portion 64a is pressed from the side close to the insertion section 12 toward the side away therefrom with the fingertip of the index finger IF, or the like. Consequently, even in a state where the press portion 64a of the second switch 64 is hard to be pressed or operated with the finger pad of the index finger IF, the function assigned to the second switch 64 can be exerted as long as the press portion can be pressed from the side close to the insertion section 12 toward the side away therefrom with the fingertip. Therefore, there can be provided the endoscope 10 having the switch 64 that is operable also in a state where the index finger IF does not easily reach the switch.

The third switch 66 disposed in the operation section 14 of the endoscope 10 according to this embodiment exerts the function, when the press portion 66a is pressed from the side close to the insertion section 12 toward the side away therefrom with the fingertip of the index finger IF, or the like. Consequently, even in a state where the press portion 66a of the third switch 66 is hard to be pressed or operated with the finger pad of the index finger IF, the function assigned to the third switch 66 can be exerted as long as the press portion can be pressed from the side close to the insertion section 12 toward the side away therefrom with the fingertip. Therefore, there can be provided the endoscope 10 having the switch 66 that is operable also in a state where the index finger IF does not easily reach the switch.

In the operation section 14 of the endoscope 10 according to this embodiment, the first to third switches 62, 64 and 66 are present at the positions at which the switches are operable with the fingertip or the finger pad of the index finger IF by moving the index finger IF in the state of holding the operation section 14. Consequently, even when the operator does not rehold the operation section 14, each of the press portions 62a, 64a and 66a of the first to third switches 62, 64 and 66 is operable with the fingertip or the finger pad of the index finger IF. Consequently, when the index finger is moved from a state where the index finger IF is placed on, for example, the fluid control button 46a or 46b or the press portion 62a of the first switch 62 to a state where the index finger IF is placed on the press portion 64a of the second switch 64 or the press portion 66a of the third switch 66, the operator does not have to rehold the operation section 14.

Furthermore, especially in the second switch 64, when the press portion 64a is pressed to change various functions or change an ON/OFF state, the press portion is pressed toward the inner portion of the operation section 14, but additionally, when the press portion is pressed from the side close to the insertion section 12 toward the side away therefrom, the function or the like is changed. Consequently, for example, even the operator who has the comparatively small hand (the comparatively short index finger IF) or even the operator who has the comparatively large hand to operate the second switch 64 while suitably performing the operation with the suitable left hand LH, the operator can suitably operate the second switch 64 without reholding the operation section 14.

Also in the third switch 66, similarly to the second switch 64, when the press portion 66a is pressed to change various functions or change an ON/OFF state, the press portion is pressed toward the inner portion of the operation section 14, but additionally, when the press portion is pressed from the side close to the insertion section 12 toward the side away therefrom, the function or the like is changed. Consequently, for example, even the operator who has the comparatively small hand (the comparatively short index finger IF) or even the operator who has the comparatively large hand to operate the third switch 66 while suitably performing the operation with the suitable left hand LH, the operator can suitably operate the second switch 64 without reholding the operation section 14.

The height (the position) of the press portion 64a of the second switch 64 is different from that of the press portion 66a of the third switch 66. Consequently, when searching for, for example, the second switch 64, the operator can place the finger pad or touch the fingertip of the index finger IF onto the press portion 64a of the second switch 64 without visually confirming the second switch 64. Similarly, when searching for the third switch 66, the operator can place the finger pad or touch the fingertip of the index finger IF onto the press portion 66a of the third switch 66 without visually confirming the third switch 66.

In this embodiment, it has been described that the same type of switches are preferably used in the second and third switches 64 and 66, but needless to say, switches different in shape including a size of the press portion 64a or 66a may be used. Also in this case, it is preferable to make a difference in height between the press portions 64a and 66a of the second and third switches 64 and 66, in that the operator's wrong operation is prevented.

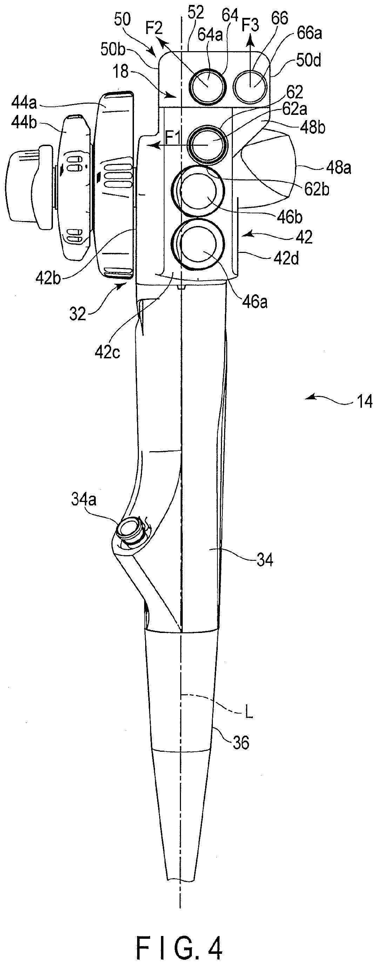

In this embodiment, it has been described that the pressing direction of the first to third switches 62, 64 and 66 is a direction in which the switch can be pressed toward the inner portion of the operation section 14. Additionally, as shown in FIG. 4, the press portions 62a, 64a and 66a of the respective switches 62, 64 and 66 may be constituted so that each press portion can be pressed only from a predetermined pressing direction. In this case, the press portion 62a of the first switch 62 can be changed, for example, by pressing the press portion in a direction F1 from the third surface 42c toward the second surface 42b in the peripheral direction of the periaxial direction of the longitudinal axis L. The press portion 64a of the second switch 64 can be changed, for example, by pressing the press portion in the same direction F2 as the direction shown in FIG. 3A. The press portion 66a of the third switch 66 can be changed, for example, by pressing the press portion from the side close to the insertion section 12 in a direction F3 away therefrom along the longitudinal axis L. That is, the press portions 62a, 64a and 66a of the first to third switches 62, 64 and 66 are different from one another in pressable direction. The first to third switches 62, 64 and 66 are formed in this manner, so that the wrong operation can be prevented as much as possible.

In this embodiment, there has been described the example where the first switch 62 is disposed in the first outer case 42 of the main body 32 of the operation section 14. Needless to say, the first switch 62 does not have to be disposed in the first outer case 42. In this case, it is preferable that, for example, the second switch 64 disposed in the second outer case 50 as described above is used as the first switch and the third switch 66 mentioned above is used as the second switch.

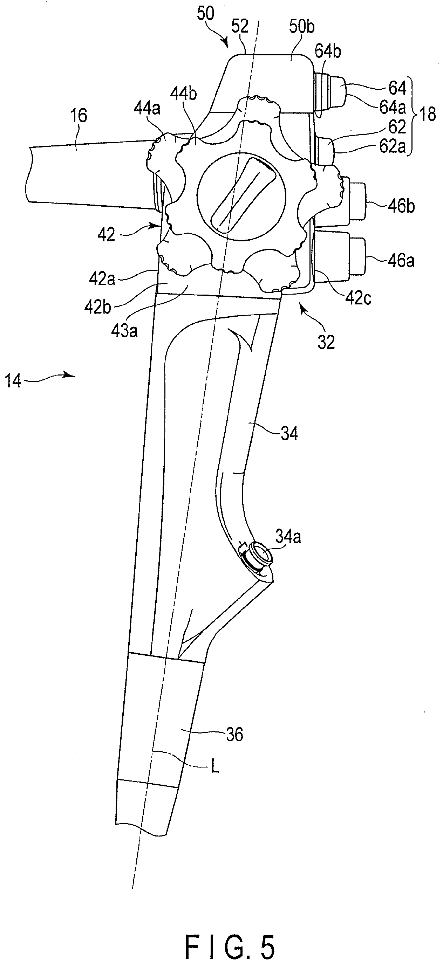

As shown in FIG. 5, it is preferable that the press portion 64a of the second switch 64 is formed to be higher than the press portion 62a of the first switch 62 in a direction perpendicular to the longitudinal axis L of the operation section 14. In this case, it is preferable that the press portion 64a of the second switch 64 projects, for example, as much as about the half of the thickness of the index finger IF, more specifically as much as approximately 4 mm to approximately 8 mm that is the about the half of the thickness (from about 9.9 mm to 15.5 mm) of the index finger IF, as compared with the press portion 62a of the first switch 62. In this way, when the difference in height between the press portions 62a and 64a of the first and second switches 62 and 64 is about the half of the thickness of the index finger IF, it is possible to prevent the wrong operations of the first and second switches 62 and 64 even in a case where the press portions 62a and 64a of the first and second switches 62 and 64 are disposed close to each other. In this case, it is preferable that the heights of the press portions 62a and 66a of the first switch 62 and the third switch 66 are about the same. At this time, without visually confirming the first to third switches 62, 64 and 66, it is possible to recognize the positions of the respective switches 62, 64 and 66. Especially, the height of the second switch 64 disposed on an upper side than the first switch 62 along the longitudinal axis L, in the direction perpendicular to the longitudinal axis L is adjusted to be larger than the height of the first switch 62 just beside the universal cord 16 (on an opposite side via the longitudinal axis L in FIG. 5) in the direction perpendicular to the longitudinal axis L. Consequently, the operator does not have to bend the fingertip in operating the second switch 64 disposed on an upper side than the universal cord 16, and an operability of the second switch 64 can improve.

It is to be noted that in FIG. 5, the second switch 64 is formed to be lower than the fluid control buttons 46a and 46b in the direction perpendicular to the longitudinal axis L. That is, in the direction perpendicular to the longitudinal axis L in FIG. 5, it is drawn that the button 46a or the button 46b is highest, the second switch 64 is next high, and the first switch 62 is lowest. Although not shown in the drawing, the height of the button 64 may be between the height of the button 46a and the height of the button 46b in the direction perpendicular to the longitudinal axis L. Consequently, the second switch 64 may be formed to be lower than at least one of the fluid control buttons 46a and 46b in the direction perpendicular to the longitudinal axis L.

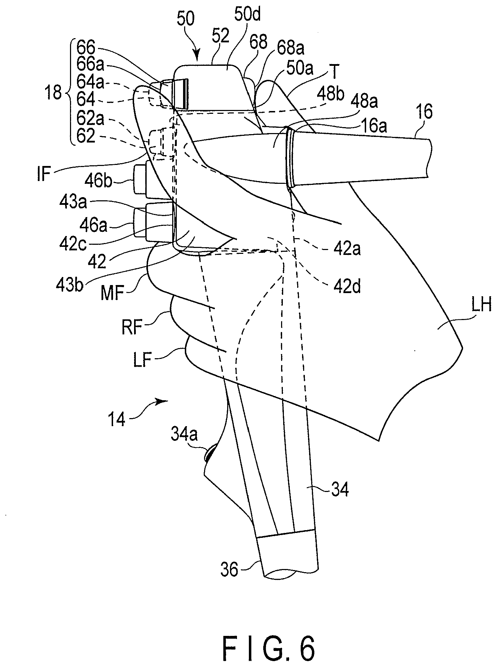

As shown in FIG. 6, needless to say, a fourth switch 68 may be disposed in the first continuous surface 50a of the second outer case 50. It is also preferable that the switch section 18 has the fourth switch 68 in the first continuous surface (the surface that is continuous with the first surface 42a along the longitudinal axis L of the operation section 14) 50a opposite to the second and third switches 64 and 66. A press portion 68a of the fourth switch 68 is pressed toward the inner portion of the operation section 14 with the finger pad of the thumb T of the operator's left hand LH. To the fourth switch 68, there is assigned a suitable function such as a function of controlling the illumination optical system to suitably select a light source and changing observation with white light and the narrow band light observation. It is also preferable that a function of controlling the observation optical system, e.g., a release function is assigned to the fourth switch 68.

### [Second Embodiment]

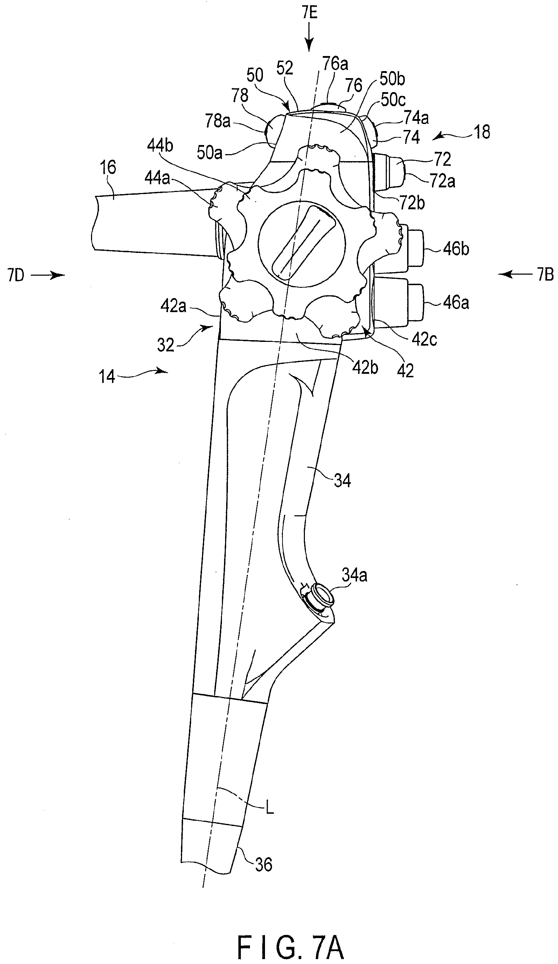

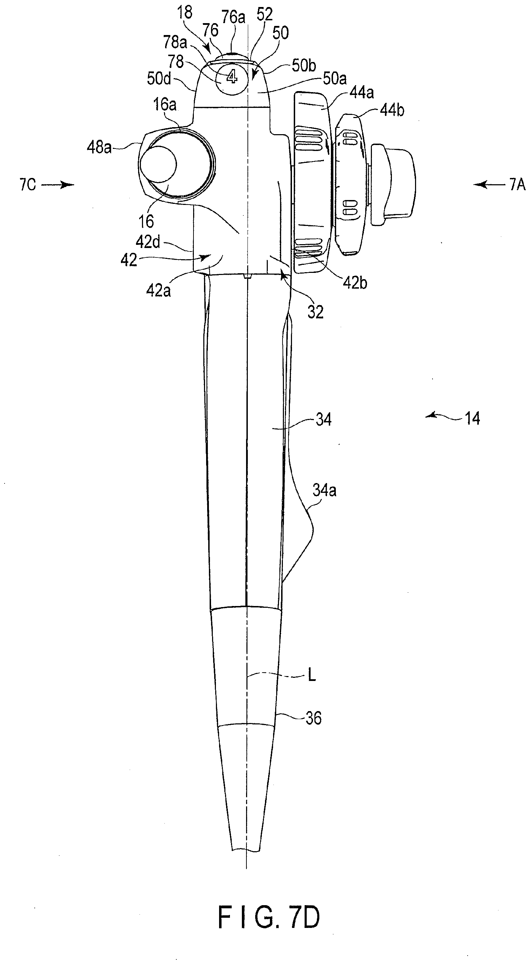

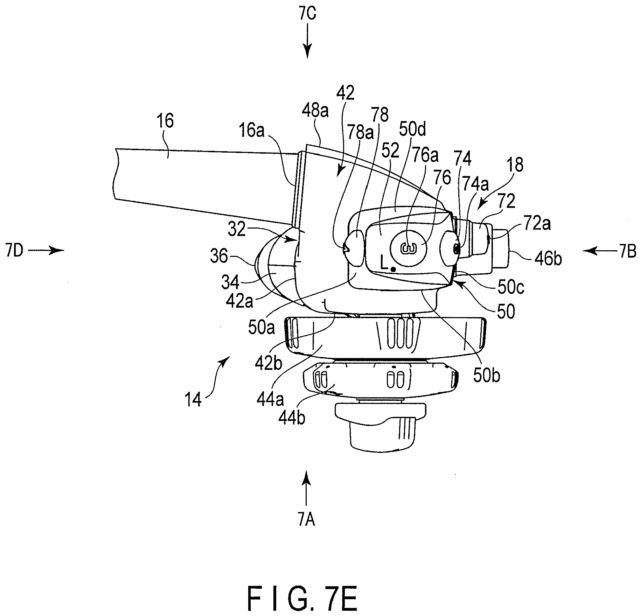

Next, a second embodiment will be described with reference to FIG. 7A to FIG. 7E. This embodiment is a modification of the first embodiment, the same members or members having the same functions as in the members described in the first embodiment are denoted with the same reference signs to the utmost, and detailed description thereof is omitted.

As shown in FIG. 7A to FIG. 7E, an endoscope 10 according to this embodiment is different from the first embodiment in first and second outer cases 42 and 50 of a main body 32 of an operation section 14 and a switch section 18.

The first bulging portion 48a is formed in the fourth surface 42d of the first outer case 42. On the other hand, in the fourth surface 42d of the first outer case 42, the second bulging portion 48b (see FIG. 1A to FIG. 1E) is not formed. The second bulging portion 48b is not formed, and accordingly a shape of a fourth continuous surface 50d of the second outer case 50 is different from that of the first embodiment.

The switch section 18 according to this embodiment includes first to fourth switches 72, 74, 76, and 78.

The first switch 72 is formed to be sandwiched between the third surface 42c of the first outer case 42 and the third continuous surface 50c of the second outer case 50. The first switch 72 may be disposed in the third surface 42c of the first outer case 42 and may be disposed in the third continuous surface 50c of the second outer case 50. The second switch 74 is disposed in the third continuous surface 50c of the second outer case 50. The third switch 76 is disposed in the end face 52 of the second outer case 50. The fourth switch 78 is disposed in the first continuous surface 50a of the second outer case 50.

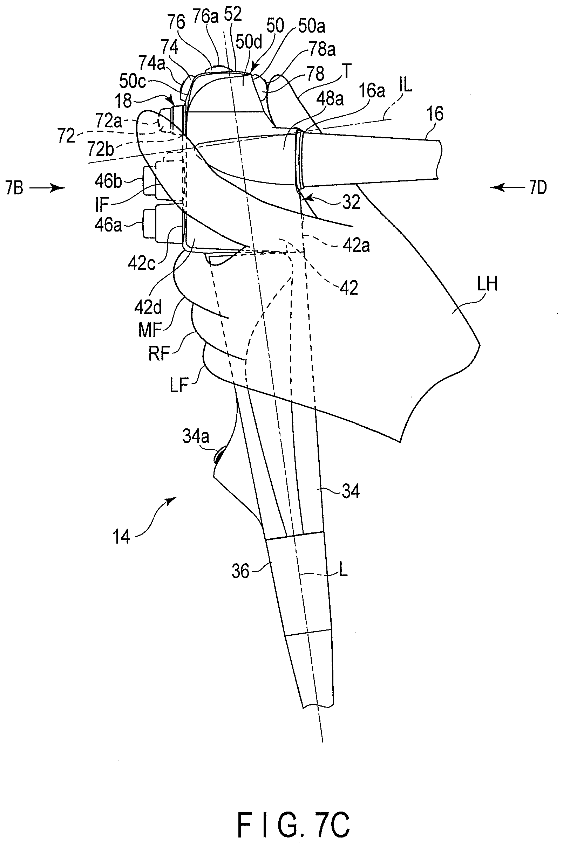

As shown in FIG. 7C, in the first switch 72, an edge portion 72b closest to an insertion section 12 along a longitudinal axis L of the operation section 14 is disposed on a side of the operation section 14 which is more away from the insertion section 12 than an imaginary line IL, on the basis of the imaginary line IL, i.e., a position of a universal cord 16 which is away from the insertion section 12 along the longitudinal axis L of the operation section 14.

As the first switch 72, a suitable switch is used so that a press portion 72a is pressed to suitably change a function. In the first switch 72, the press portion 72a is pressed toward an inner portion of the operation section 14 to suitably change the function. In the first switch 72, the press portion 72a is pressed from a side close to the insertion section 12 toward a side away therefrom to suitably change the function. Alternatively, in the first switch 72, the press portion 72a is pressed from the side close to the insertion section 12 toward the side away therefrom and in a periaxial direction of the longitudinal axis L of the operation section 14 to suitably change the function. In this way, the first switch 72 can be pushed from the side close to the insertion section 12 toward the side away therefrom. Especially, the first switch 72 can be pushed from the side close to the insertion section 12 toward the side away therefrom and from the fourth continuous surface 50d toward a second continuous surface 50b in the periaxial direction of the longitudinal axis L of the operation section 14.

Consequently, in the first switch 72, the press portion 72a can be pressed toward the inner portion of the operation section 14 with the finger pad of the index finger IF of the operator's left hand LH.

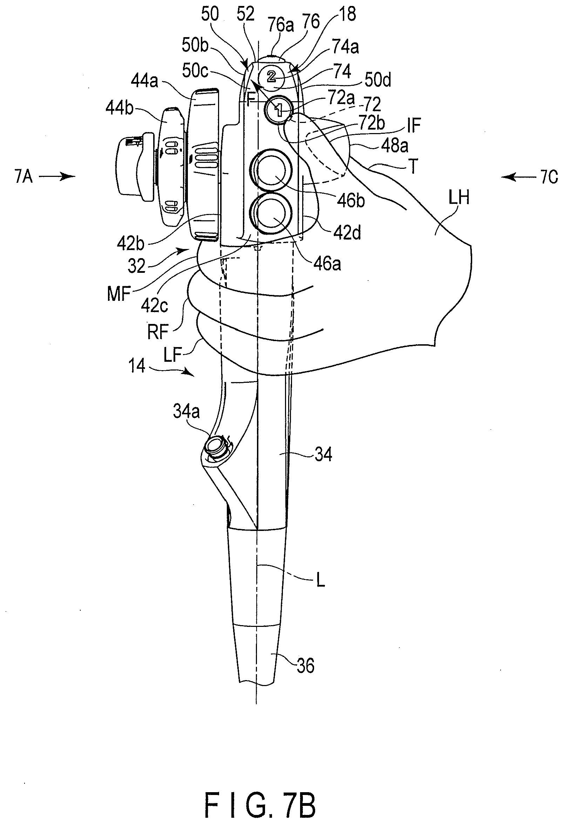

When it is difficult for the operator to extend the index finger IF, the operator may give a force to the press portion 72a of the first switch 72 in a direction of an arrow F in FIG. 7B with the fingertip of the index finger IF. In the first switch 72, the press portion 72a can be pressed from the side close to the insertion section 12 toward the side away therefrom with, for example, the fingertip of the index finger IF of the operator's left hand LH. In the first switch 72, the press portion 72a can be pressed from the side close to the insertion section 12 toward the side away therefrom and in the periaxial direction of the longitudinal axis L of the operation section 14 with the index finger IF of the operator' s left hand LH. That is, the first switch 72 may be pressed in the periaxial direction of the longitudinal axis L of the operation section 14, and the force can be given to press the first switch toward the inner portion of the operation section 14. Consequently, the operator operates the press portion 72a of the first switch 72 in various directions, whereby the function of the first switch 72 can be exerted.

In the second and third switches 74 and 76, press portions 74a and 76a can be pressed toward the inner portion of the operation section 14 with the finger pad of the operator's index finger. In the fourth switch 78, a press portion 78a can be pressed toward the inner portion of the operation section 14 with the finger pad of an operator's thumb.

A normal direction of the third continuous surface 50c in which the second switch 74 is disposed is in a direction away from the insertion section 12 with respect to a normal direction of the third surface 42c. Consequently, the second switch 74 is formed so that the press portion 74a is pressed toward an inner portion of the second outer case 50, i.e., toward the inner portion of the operation section 14, thereby suitably changing a function of an observation optical system or an illumination optical system.

Additionally, in the second switch 74, it is preferable that the operator who has comparatively small hands operates the press portion 74a of the second switch 74 from various directions in the same manner as in the first switch 72, whereby a function of the second switch 74 can be exerted. That is, in the second switch 74, it is preferable that not only when the press portion 74a is pressed toward the inner portion of the operation section 14 with the finger pad of the index finger IF of the operator' s left hand LH but also when the press portion 74a is pressed from the side close to the insertion section 12 toward the side away therefrom with, for example, the fingertip of the index finger IF of the operator's left hand LH, the function of the second switch 74 can be exerted. Furthermore, in the second switch 74, it is preferable that the press portion 74a can be pressed from the side close to the insertion section 12 toward the side away therefrom and in the periaxial direction of the longitudinal axis L of the operation section 14 with the index finger IF of the operator's left hand LH. Therefore, there can be provided the endoscope 10 having the switch 74 that is operable also in a state where it is difficult for the operator' s index finger IF to reach the switch.

A normal direction of the end face 52 of the second outer case 50 in which the third switch 76 is disposed extends, for example, outwardly from the operation section 14 along the longitudinal axis L of the operation section 14. Consequently, the third switch 76 is formed so that the press portion 76a is pressed toward the inner portion of the second outer case 50, i.e., toward the inner portion of the operation section 14, thereby suitably changing the function of the observation optical system or the illumination optical system.

A normal direction of the first continuous surface 50a in which the fourth switch 78 is disposed is in a direction away from the insertion section 12 with respect to the normal direction of the first surface 42a. Consequently, the fourth switch 78 is formed so that the press portion 78a is pressed toward the inner portion of the second outer case 50, i.e., toward the inner portion of the operation section 14, thereby suitably changing the function of the observation optical system or the illumination optical system.

It is to be noted that in the second switch 74, it is possible to operate the press portion 74a with the index finger IF without reholding the operation section 14. Furthermore, the fourth switch 78 can suitably be operated with the thumb T. Consequently, the operator can suitably operate the first, second and fourth switches 72, 74 and 78 without reholding the operation section 14.

The first switch 72 is pressed from the side close to the insertion section 12 toward the side away therefrom with the fingertip of the index finger IF, whereby the function of the observation optical system or the illumination optical system can suitably be changed. Consequently, also in a case where the operator who has comparatively small hands operates the first switch 72 with the index finger IF or in a case where various operations of the operation section 14 are performed with a thumb or a finger other than the index finger IF and hence it is difficult for the finger pad of the index finger IF to reach the switch, it is possible to suitably operate the first switch 72. Therefore, there can be provided the endoscope 10 having the switch 72 that is operable also in a state where the index finger IF does not easily reach the switch.

The bulging portion 48a formed in the fourth surface 42d of the first outer case 42 interferes when the operator extends the index finger of the left hand LH toward the third switch 76. Consequently, in a case of operating the third switch 76, the operator reholds the operation section 14 and then presses the press portion 76a toward the inner portion of the operation section 14. Thus, it is preferable that to the third switch 76, there is assigned a function that is less changed in the observation optical system and the illumination optical system or a function that is less performed while performing another operation such as an operation of a turning knob 44a or 44b.

Although the several embodiments have been specifically described with reference to the drawings, the present invention is not restricted to the foregoing embodiments and includes all embodiments carried out without departing from the gist thereof.

### Reference Signs List

10···Endoscope, 14···Operation section, 16···Universal cord, 18···Switch section, 32···Main body, 34···Holding portion, 42··· first outer case, 42a···First surface, 42b···Second surface, 42c ···Third surface, 42d···Fourth surface, 44a, 44b···Turning knob, 46a, 46b···Fluid control button, 48a···First bulging portion, 48b ···Second bulging portion, 50···Second outer case, 50a···First continuous surface, 50b···Second continuous surface, 50c···Third continuous surface, 50d···Fourth continuous surface, 52···End face, 62···First switch, 62a···Press portion, 64···Second switch, 64a···Press portion, 66···Third switch, 66a···Press portion, L ···Longitudinal axis, LH···Left hand, IF···Index finger.

## Claims

1. An endoscope comprising:
an operation section which is connected to an insertion section to be inserted into a subject, and which is configured to operate the insertion section;
a universal cord which is connected to the operation section at one end thereof, and which is configured to connect an external device to the operation section;
a first switch which is disposed at about the same position as a connecting position to the universal cord along a longitudinal axis of the operation section; and
a second switch which is disposed on a side opposite to the insertion section via the first switch along the longitudinal axis of the operation section, and which is formed to be higher than the first switch in a direction perpendicular to the longitudinal axis.

2. The endoscope according to claim 1, further comprising:
a fluid control button which is disposed on a side closer to the insertion section than the first switch along the longitudinal axis of the operation section, and which is configured to control a fluid in the insertion section,
wherein the second switch is formed to be lower than the fluid control button in the direction perpendicular to the longitudinal axis.

3. The endoscope according to claim 1, wherein:
each of the first switch and the second switch has a press portion to be pressed, and
the press portion of the second switch is formed to be higher than the press portion of the first switch in the direction perpendicular to the longitudinal axis.

4. The endoscope according to claim 1, wherein the first switch and the second switch are different from each other in pressable direction.

5. The endoscope according to claim 1, further comprising:
a third switch which is adjacent to the second switch in a peripheral direction of the longitudinal axis,
wherein the third switch is pressable from a side close to the insertion section toward a side away therefrom.

6. The endoscope according to claim 1, wherein in the second switch, an edge portion closest to the insertion section along the longitudinal axis of the operation section is disposed on a side of the operation section which is more away from the insertion section than a position of the universal cord which is most away from the insertion section along the longitudinal axis of the operation section.

7. The endoscope according to claim 1, further comprising:
a fluid control button which is disposed on a side of the operation section which is closer to the insertion section than the first switch along the longitudinal axis to control a fluid in the insertion section,
wherein the operation section includes first and second outer cases which form an outer shell thereof,
in the first outer case, the fluid control button and the first switch are disposed,
in the second outer case, the second switch is disposed, and
the second outer case is disposed on a side more away from the insertion section than the first outer case.

8. The endoscope according to claim 7, wherein the first outer case includes a base body having a substantially C-shaped cross section of the longitudinal axis of the operation section, and a lid portion that holds the universal cord and forms a lid on the base body.
